# EUROPEAN PATENT APPLICATION

(11) **EP 4 716 250 A1**
(43) Date of publication of application: **25.03.2026**
(21) Application number: 24806873.6
(22) Date of filing: 01.04.2024
(51) Int. Cl.: H04R 1/34

(54) **ULTRASONIC WAVE GENERATION DEVICE**

(30) Priority: 17.05.2023 JP 2023081666
(71) Applicant: Niterra Co., Ltd., Nagoya-shi, Aichi 461-0005 (JP)
(72) Inventor: ITOH Shinsuke, Nagoya-shi, Aichi 461-0005 (JP); YOKOYAMA Kota, Nagoya-shi, Aichi 461-0005 (JP); SUZUKI Satoshi, Nagoya-shi, Aichi 461-0005 (JP); SUZUKI Ryo, Nagoya-shi, Aichi 461-0005 (JP); KASASHIMA Takashi, Nagoya-shi, Aichi 461-0005 (JP)
(74) Representative: Grünecker Patent- und Rechtsanwälte PartG mbB
(86) International application number: PCT/JP2024/013430
(87) International publication number: WO 2024/236919

(57) **Abstract**

An ultrasonic wave generating device (10) includes an ultrasonic wave generating source (11), an ultrasonic wave focusing part (12), and a waveguide (13). The ultrasonic wave focusing part (12) has a first reflection surface (21) and a second reflection surface (22). The first reflection surface (21) reflects ultrasonic waves generated by the ultrasonic wave generating source (11). The second reflection surface (22) reflects the ultrasonic waves reflected by the first reflection surface (21). The ultrasonic waves reflected by the second reflection surface (22) are reflected as plane waves and introduced into an introduction part (25) of the waveguide (13). The ultrasonic wave generating source (11) has a radiation surface (15). The ultrasonic wave focusing part (12) has a joining surface (20) joined to the radiation surface (15). The introduction part (25) and the second reflection surface (22) are arranged to be aligned in a front-rear direction. The joining surface (20) is arranged on one side in a first direction, which is orthogonal to the front-rear direction. The first reflection surface (21) is arranged on an other side in the first direction relative to the joining surface (20).

## Description

### Technical Field

The present disclosure relates to an ultrasonic wave generating device.

### Background Art

An ultrasonic wave generating device is disclosed in PTL 1. This ultrasonic wave generating device includes an ultrasonic wave generating source and a focusing part. The focusing part includes a first reflector that reflects at a first reflection surface thereof ultrasonic waves generated by the ultrasonic wave generating source, a second reflector that reflects at a second reflection surface thereof the ultrasonic waves reflected by the first reflection surface, and a waveguide that serves as a transmission path for the ultrasonic waves. The waveguide is disposed such that the ultrasonic waves reflected by the second reflection surface are introduced into the waveguide itself through an introduction part thereof. The focal point of the second reflection surface and the focal point of the first reflection surface are set such that the ultrasonic waves reflected by the second reflection surface are reflected as plane waves.

### Citation List

### Patent Literature

PTL 1: Japanese Unexamined Patent Application Publication No. 2021-90181

### Summary of Invention

### Technical Problem

In the configuration according to PTL 1, the ultrasonic wave generating source needs to be arranged so as to generate ultrasonic waves frontward along the axial direction. Depending on circumstances, however, such an arrangement may be unpreferable, and an alternative arrangement is desired as a new option.

The present disclosure is to provide an ultrasonic wave generating device in which an ultrasonic wave generating source is arranged in a novel configuration. Solution to Problem

An ultrasonic wave generating device according to the present disclosure is an ultrasonic wave generating device comprising:
an ultrasonic wave generating source that generates ultrasonic waves;
an ultrasonic wave focusing part that focuses the ultrasonic waves generated by the ultrasonic wave generating source; and
a waveguide that transmits the ultrasonic waves focused by the ultrasonic wave focusing part,
wherein the ultrasonic wave focusing part has
a first reflection surface that reflects the ultrasonic waves generated by the ultrasonic wave generating source; and
a second reflection surface that reflects the ultrasonic waves reflected by the first reflection surface,
wherein the first reflection surface and the second reflection surface are arranged such that the ultrasonic waves reflected by the second reflection surface are reflected as plane waves and introduced into an introduction part of the waveguide,
wherein the ultrasonic wave generating source has a radiation surface that radiates the ultrasonic waves toward the ultrasonic wave focusing part,
wherein the ultrasonic wave focusing part has a joining surface joined to the radiation surface,
wherein the introduction part and the second reflection surface are arranged to be aligned in the front-rear direction,
wherein the joining surface is arranged on one side in a first direction, the first direction being a specific direction among directions orthogonal to the front-rear direction, and
wherein the first reflection surface is arranged on an other side in the first direction relative to the joining surface.

### Advantageous Effects of Invention

According to the present disclosure, an ultrasonic wave generating device in which an ultrasonic wave generating source is arranged in a novel configuration can be provided.

### Brief Description of Drawings

[Fig. 1] Fig. 1 is a perspective view of an ultrasonic wave generating device according to a first embodiment.
[Fig. 2] Fig. 2 is a sectional view of the ultrasonic wave generating device according to the first embodiment.
[Fig. 3] Fig. 3 is a sectional view of an ultrasonic wave generating device according to a comparative example.
[Fig. 4] Fig. 4 is a sectional view of an ultrasonic wave generating device according to a second embodiment.
[Fig. 5] Fig. 5 is a sectional view of an ultrasonic wave generating device according to a third embodiment.
[Fig. 6] Fig. 6 is a sectional view of an ultrasonic wave generating device according to a fourth embodiment.
[Fig. 7] Fig. 7 is a sectional view of an ultrasonic wave generating device according to a fifth embodiment.
[Fig. 8] Fig. 8 is a sectional view of an ultrasonic wave generating device according to a sixth embodiment.
[Fig. 9] Fig. 9 is a sectional view of an ultrasonic wave generating device according to a seventh embodiment.
[Fig. 10] Fig. 10 is a sectional view of an ultrasonic wave generating device according to an eighth embodiment.
[Fig. 11] Fig. 11 is a diagram illustrating another example of a waveguide.

### Description of Embodiments

### [Description of Embodiments of Disclosure]

Embodiments of the present disclosure are listed and exemplified below.

[1] An ultrasonic wave generating device comprising:
   an ultrasonic wave generating source that generates ultrasonic waves;
   an ultrasonic wave focusing part that focuses the ultrasonic waves generated by the ultrasonic wave generating source; and
   a waveguide that transmits the ultrasonic waves focused by the ultrasonic wave focusing part,
   wherein the ultrasonic wave focusing part has
   a first reflection surface that reflects the ultrasonic waves generated by the ultrasonic wave generating source; and
   a second reflection surface that reflects the ultrasonic waves reflected by the first reflection surface,
   wherein the first reflection surface and the second reflection surface are arranged such that the ultrasonic waves reflected by the second reflection surface are reflected as plane waves and introduced into an introduction part of the waveguide,
   wherein the ultrasonic wave generating source has a radiation surface that radiates the ultrasonic waves toward the ultrasonic wave focusing part,
   wherein the ultrasonic wave focusing part has a joining surface joined to the radiation surface,
   wherein the introduction part and the second reflection surface are arranged to be aligned in the front-rear direction,
   wherein the joining surface is arranged on one side in a first direction, the first direction being a specific direction among directions orthogonal to the front-rear direction, and
   wherein the first reflection surface is arranged on an other side in the first direction relative to the joining surface.

In the above ultrasonic wave generating device, the joining surface is arranged on one side in the first direction that is orthogonal to the front-rear direction, and the first reflection surface is arranged on the other side in the first direction relative to the joining surface. The ultrasonic wave generating source is joined to the joining surface thus arranged. That is, in the above ultrasonic wave generating device, the ultrasonic wave generating source can be arranged in a novel configuration.

[2] The ultrasonic wave generating device according to [1],
wherein the waveguide has a shape extending in a second direction, the second direction being orthogonal to both the front-rear direction and the first direction.

In the above ultrasonic wave generating device, the ultrasonic waves focused by the ultrasonic wave focusing part can be transmitted over a wide range in the second direction.

[3] The ultrasonic wave generating device according to [1],
wherein a plurality of said waveguides are arranged at intervals from one another in a second direction, the second direction being orthogonal to both the front-rear direction and the first direction.

In the above ultrasonic wave generating device, the ultrasonic waves focused by the ultrasonic wave focusing part can be transmitted through each of the plurality of waveguides arranged at intervals in the second direction.

[4] The ultrasonic wave generating device according to any of [1] to [3],
wherein a flow path is provided inside the waveguide.

In the above ultrasonic wave generating device, liquid flows through the flow path, and the ultrasonic waves are transmitted through the liquid, whereby the efficiency of transmission of the ultrasonic waves can be improved.

[5] The ultrasonic wave generating device according to any of [1] to [4],
wherein the waveguide has a hollow, and
wherein the hollow is filled with liquid.

In the above ultrasonic wave generating device, the ultrasonic waves are transmitted through the liquid filling the hollow, whereby the efficiency of transmission of the ultrasonic waves can be improved.

[6] The ultrasonic wave generating device according to any of [1] to [5],
wherein the joining surface and the first reflection surface are arranged such that the ultrasonic waves radiated from the radiation surface travel through an area between the introduction part and the second reflection surface.

The energy loss occurring at the reflection of the ultrasonic waves varies with the angle of incidence and the angle of reflection. In a known configuration such as the one disclosed by PTL 1, in the case of ultrasonic waves generated from a radially outer position of the ultrasonic wave generating source, the angle of incidence and the angle of reflection thereof are large when reflected at the first reflection surface, and are also large when reflected at the second reflection surface. On the other hand, in the case of ultrasonic waves generated from a radially inner position of the ultrasonic wave generating source, the angle of incidence and the angle of reflection thereof are small when reflected at the first reflection surface, and are also small when reflected at the second reflection surface. Therefore, the energy of the ultrasonic waves introduced into the waveguide tends to vary.

In contrast, in the above ultrasonic wave generating device, when the angle of incidence and the angle of reflection when reflected at the first reflection surface are large, the angle of incidence and the angle of reflection when reflected at the second reflection surface are small. Conversely, when the angle of incidence and the angle of reflection when reflected at the first reflection surface are small, the angle of incidence and the angle of reflection when reflected at the second reflection surface are large. Therefore, the energy of the ultrasonic waves introduced into the waveguide is less likely to vary.

[7] The ultrasonic wave generating device according to any of [1] to [6],
wherein the radiation surface is formed on a surface of the ultrasonic wave generating source, the surface being located on one side in a thickness direction of the ultrasonic wave generating source, and
wherein a thickness of the ultrasonic wave generating source is smaller than a width of the ultrasonic wave generating source.

In the above ultrasonic wave generating device, the joining surface is arranged on one side in the first direction that is orthogonal to the front-rear direction, and the first reflection surface is arranged on the other side in the first direction relative to the joining surface. The ultrasonic wave generating source is joined to the joining surface thus arranged. Accordingly, the ultrasonic wave generating source is arranged such that the width direction of the ultrasonic wave generating source is slanted or orthogonal with respect to the first direction. Since the thickness of the ultrasonic wave generating source is smaller than the width of the ultrasonic wave generating source, the above ultrasonic wave generating device is more easily reduced in size in the first direction as compared with the known configuration in which the width direction of the ultrasonic wave generating source is parallel to the first direction.

### [Details of Embodiments of Disclosure]

The ultrasonic wave generating device according to the present disclosure is intended for, for example, ultrasonic diagnosis apparatuses, ultrasonic therapy apparatuses, cavitation generating apparatuses, dental scalers, blood coagulation surgery apparatuses (such as ultrasonic scalpels), ultrasonic processing machines, ultrasonic washing machines (such as ultrasonic cleaners), and so forth.

### 1. First Embodiment

### 1-1. Configuration of Ultrasonic Wave Generating Device 10

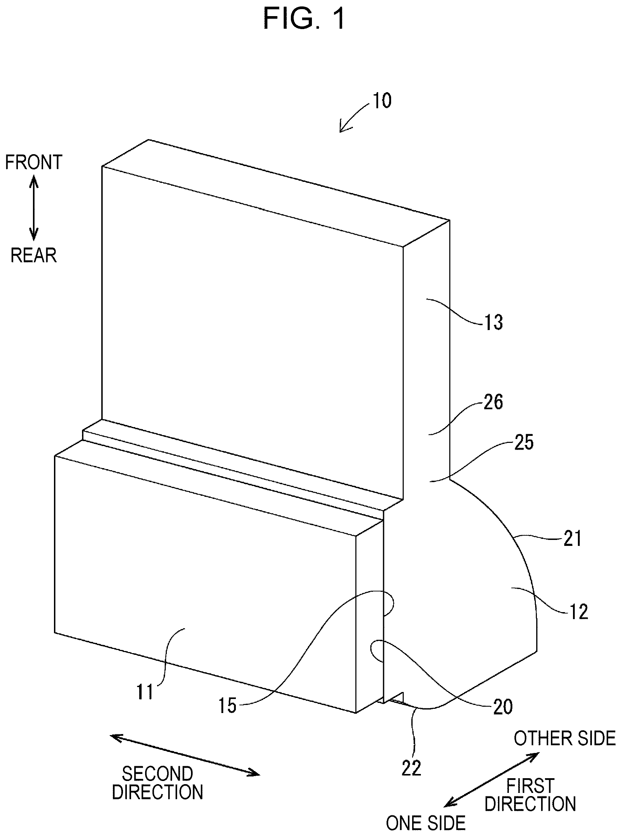

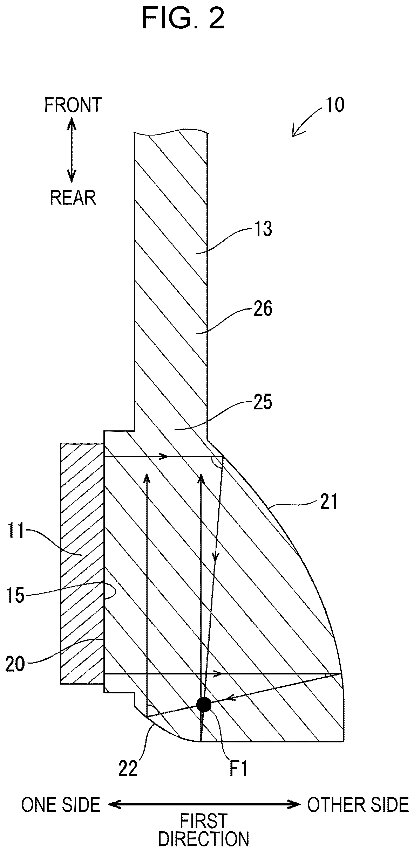

As illustrated in Figs. 1 and 2, an ultrasonic wave generating device 10 includes an ultrasonic wave generating source 11, an ultrasonic wave focusing part 12, and a waveguide 13. The ultrasonic wave generating source 11 generates ultrasonic waves. The ultrasonic wave focusing part 12 focuses the ultrasonic waves generated by the ultrasonic wave generating source 11. The waveguide 13 transmits the ultrasonic waves focused by the ultrasonic wave focusing part 12.

The ultrasonic wave generating source 11 is, for example, a piezo-electric element. The piezo-electric element includes a piezo-electric body made of piezo ceramic, and electrodes disposed on two sides of the piezo-electric body. The electrodes are provided on both sides in the thickness direction of the ultrasonic wave generating source 11. The thickness of the ultrasonic wave generating source 11 is smaller than the width of the ultrasonic wave generating source 11.

When the ultrasonic wave generating source 11 receives an electric signal from the signal transmitter-receiver circuit not illustrated, the ultrasonic wave generating source 11 generates ultrasonic waves. The ultrasonic wave generating source 11 generates the ultrasonic waves at a frequency of, for example, 30 kHz or higher and 10 MHz or lower. The ultrasonic wave generating source 11 has a radiation surface 15, which radiates the ultrasonic waves toward the ultrasonic wave focusing part 12. The radiation surface 15 is a flat surface. The ultrasonic waves travel from the radiation surface 15 of the ultrasonic wave generating source 11 in a direction perpendicular to the radiation surface 15. The radiation surface 15 is formed on a surface of the ultrasonic wave generating source 11 that is located on one side in the thickness direction of the ultrasonic wave generating source 11. The radiation surface 15 is joined to the ultrasonic wave focusing part 12.

The ultrasonic wave focusing part 12 is made of metal (such as duralumin). The ultrasonic wave focusing part 12 has a joining surface 20, which is joined to the ultrasonic wave generating source 11 (more specifically, to the radiation surface 15).

The ultrasonic wave focusing part 12 has a first reflection surface 21 and a second reflection surface 22. The first reflection surface 21 reflects toward the second reflection surface 22 the ultrasonic waves generated by the ultrasonic wave generating source 11. The second reflection surface 22 reflects toward an introduction part 25 of the waveguide 13 the ultrasonic waves reflected by the first reflection surface 21. The first reflection surface 21 and the second reflection surface 22 are arranged such that the ultrasonic waves reflected by the second reflection surface 22 are reflected as plane waves and introduced into the introduction part 25 of the waveguide 13.

The waveguide 13 may be the same member as the ultrasonic focusing part 12, or may be a separate member from the ultrasonic focusing part 12. The waveguide 13 may preferably be made of a material exhibiting a high propagation capability for ultrasonic waves, and preferable examples of the material include an aluminum alloy, metal glass, and the like. The waveguide 13 may alternatively be made of, for example, a shape-memory alloy composed of titanium and nickel. The waveguide 13 is elastically deformable. The waveguide 13 includes an extended part 26, which extends frontward from the ultrasonic wave focusing part 12. The size and shape of the cross section of the extended part 26 are constant in the front-rear direction. A proximal portion of the extended part 26 serves as the introduction part 25. The direction in which the extended part 26 extends may be parallel to the front-rear direction or may be slightly slanted with respect to the front-rear direction.

The introduction part 25 and the second reflection surface 22 are arranged to be aligned in the front-rear direction. The introduction part 25 is arranged on the front side relative to the second reflection surface 22. When seen from the outside of the ultrasonic wave focusing part 12, the second reflection surface 22 is a curved surface (such as a paraboloid) that is convex toward the rear side (the side opposite to the second reflection surface 22). When seen from the inside of the ultrasonic wave focusing part 12, the second reflection surface 22 has a concave shape. The plane waves generated at the second reflection surface travel frontward toward the introduction part 25. That is, the traveling direction of the plane waves generated at the second reflection surface is a frontward direction.

The joining surface 20 described above is arranged on one side of the ultrasonic wave focusing part 12 in a first direction. The first direction is a specific direction among directions orthogonal to the front-rear direction. The joining surface 20 is arranged along the front-rear direction. The radiation surface 15 of the ultrasonic wave generating source 11 is joined to the joining surface 20. The radiation surface 15 is arranged along the front-rear direction. The thickness direction of the ultrasonic wave generating source 11 is parallel to the first direction. The radiation surface 15 is arranged facing the first reflection surface 21 of the ultrasonic wave focusing part 12. The first reflection surface 21 is arranged on an other side in the first direction relative to the joining surface 20. When seen from the outside of the ultrasonic wave focusing part 12, the first reflection surface 21 is a curved surface (such as a paraboloid) that is convex toward the other side in the first direction. The first reflection surface 21 curves rearward while extending toward the other side in the first direction. When seen from the inside of the ultrasonic wave focusing part 12, the first reflection surface 21 has a concave shape.

The joining surface 20 is arranged on the one side in the first direction relative to the introduction part 25 and the second reflection surface 22. The first reflection surface 21 is arranged on the other side in the first direction relative to the introduction part 25 and the second reflection surface 22. In the front-rear direction, the joining surface 20 and the first reflection surface 21 are arranged between the second reflection surface 22 and the introduction part 25. The joining surface 20 and the first reflection surface 21 are arranged such that the ultrasonic waves radiated from the radiation surface 15 travel through an area between the introduction part 25 and the second reflection surface.

When the ultrasonic wave generating source 11 receives an electric signal from the signal transmitter-receiver circuit not illustrated, the ultrasonic wave generating source 11 generates ultrasonic waves from the radiation surface 15 thereof toward the first reflection surface 21 as illustrated in Fig. 2. The ultrasonic waves radiated from the radiation surface 15 are reflected by the first reflection surface 21 and are focused toward a focal point F1 of the first reflection surface 21. The focal point F1 of the first reflection surface 21 is the same as that of the second reflection surface 22. Therefore, the ultrasonic waves having traveled through the focal point F1 of the first reflection surface 21 are reflected by the second reflection surface 22 and introduced as plane waves into the waveguide 13.

As illustrated in Fig. 1, the ultrasonic wave generating source 11, the ultrasonic wave focusing part 12, and the waveguide 13 each have a shape extending in a second direction, which is orthogonal to both the front-rear direction and the first direction.

The radiation surface 15, the first reflection surface 21, the second reflection surface 22, and the introduction part 25 are each arranged to be continuous along the second direction. The radiation surface 15 radiates ultrasonic waves over a wide range in the second direction. The first reflection surface 21 reflects over a wide range in the second direction the ultrasonic waves radiated by the radiation surface 15. The second reflection surface 22 reflects over a wide range in the second direction the ultrasonic waves reflected by the first reflection surface 21. The ultrasonic waves reflected by the second reflection surface 22 are introduced over a wide range in the second direction into the introduction part 25. The waveguide 13 transmits frontward the ultrasonic waves introduced from the introduction part 25.

### 1-2. Exemplary Effects of Ultrasonic Wave Generating Device 10

In the ultrasonic wave generating device 10, the joining surface 20 is arranged on one side in the first direction that is orthogonal to the front-rear direction, whereas the first reflection surface 21 is arranged on the other side in the first direction relative to the joining surface 20. The ultrasonic wave generating source 11 is joined to the joining surface 20 thus arranged. That is, in the ultrasonic wave generating device 10, the ultrasonic wave generating source 11 can be arranged in a novel configuration.

In the ultrasonic wave generating device 10, the ultrasonic waves focused by the ultrasonic wave focusing part 12 can be transmitted over a wide range in the second direction.

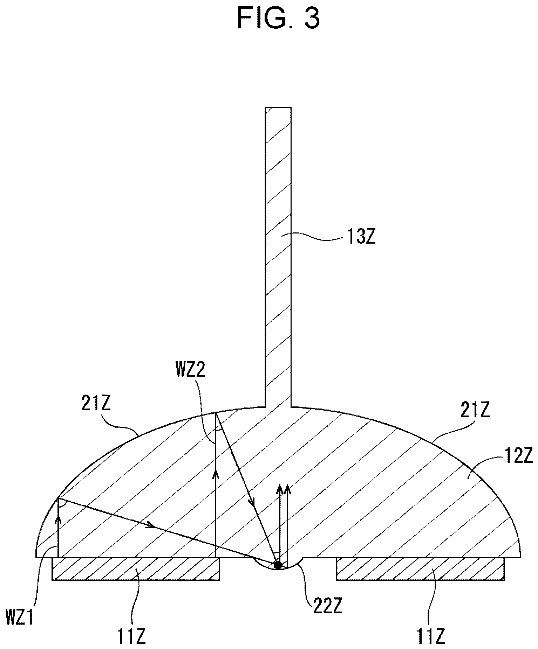

The energy loss occurring at the reflection of the ultrasonic waves varies with the angle of incidence and the angle of reflection. In a known configuration such as the one illustrated in Fig. 3, in the case of ultrasonic waves WZ1 generated from a radially outer position of an ultrasonic wave generating source 11Z, the angle of incidence and the angle of reflection thereof are large when reflected at a first reflection surface 21Z of an ultrasonic wave focusing part 12Z, and are also large when reflected at a second reflection surface. On the other hand, in the case of ultrasonic waves WZ2 generated from a radially inner position of the ultrasonic wave generating source 11Z, the angle of incidence and the angle of reflection thereof are small when reflected at the first reflection surface 21Z, and are also small when reflected at the second reflection surface 22Z. Therefore, the energy of the ultrasonic waves introduced into a waveguide 13Z tends to vary.

In contrast, in the ultrasonic wave generating device 10 illustrated in Fig. 2, when the angle of incidence and the angle of reflection when reflected at the first reflection surface 21 are large, the angle of incidence and the angle of reflection when reflected at the second reflection surface 22 are small. Conversely, when the angle of incidence and the angle of reflection when reflected at the first reflection surface 21 are small, the angle of incidence and the angle of reflection when reflected at the second reflection surface 22 are large. Therefore, the energy of the ultrasonic waves introduced into the waveguide 13 is less likely to vary.

In the ultrasonic wave generating device 10, the joining surface 20 is arranged on one side in the first direction that is orthogonal to the front-rear direction, and the first reflection surface 21 is arranged on the other side in the first direction relative to the joining surface 20. The ultrasonic wave generating source 11 is joined to the joining surface 20 thus arranged. Accordingly, the ultrasonic wave generating source 11 is arranged such that the width direction of the ultrasonic wave generating source 11 is slanted or orthogonal with respect to the first direction. Since the thickness of the ultrasonic wave generating source 11 is smaller than the width of the ultrasonic wave generating source 11, the ultrasonic wave generating device 10 is more easily reduced in size in the first direction as compared with the known configuration (see Fig. 3) in which the width direction of the ultrasonic wave generating source 11 is parallel to the first direction.

### 2. Second Embodiment

A second embodiment describes a configuration in which the ultrasonic waves reflected by the first reflection surface are reflected by the second reflection surface before reaching the focal point.

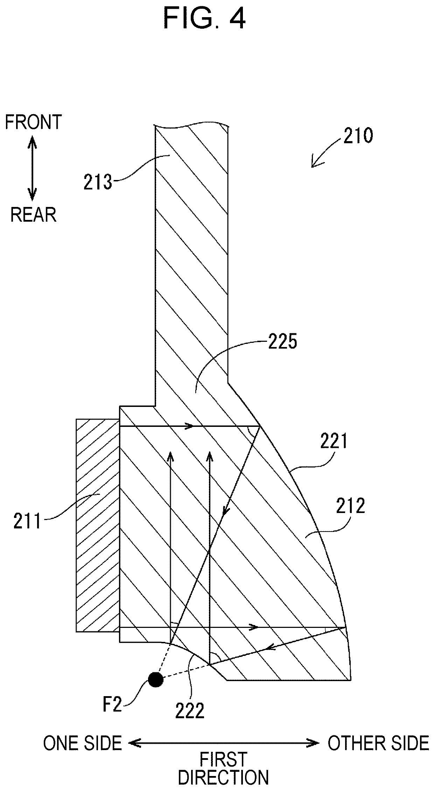

As illustrated in Fig. 4, an ultrasonic wave generating device 210 according to the second embodiment includes an ultrasonic wave generating source 211, an ultrasonic wave focusing part 212, and a waveguide 213. The ultrasonic wave focusing part 212 has a first reflection surface 221 and a second reflection surface 222.

The ultrasonic wave generating device 210 is different from the ultrasonic wave generating device 10 according to the first embodiment mainly in having the second reflection surface 222 in replacement of the second reflection surface 22. The other elements of the ultrasonic wave generating device 210, excluding the second reflection surface 222, are the same in function as those of the first embodiment, with slight adjustments due to the provision of the second reflection surface 222 to the ultrasonic wave generating device 210.

When seen from the outside of the ultrasonic wave focusing part 12, the second reflection surface 222 is a curved surface (such as a paraboloid) that is concave frontward (toward the first reflection surface 221). When seen from the inside of the ultrasonic wave focusing part 212, the second reflection surface 222 has a convex shape. A focal point F2 of the second reflection surface 222 is the same as that of the first reflection surface 221. The second reflection surface 222 is arranged closer to the first reflection surface 221 than the focal point F2 of the first reflection surface 221 is.

The first reflection surface 221 reflects toward the second reflection surface 222 the ultrasonic waves generated by the ultrasonic wave generating source 211. The second reflection surface 222 reflects toward an introduction part 225 of the waveguide 213 the ultrasonic waves reflected by the first reflection surface 221. The first reflection surface 221 and the second reflection surface 222 are arranged such that the ultrasonic waves reflected by the second reflection surface 222 are reflected as plane waves and introduced into the introduction part 225 of the waveguide 213.

The ultrasonic wave generating device 210 according to the second embodiment can cause the ultrasonic waves generated by the ultrasonic wave generating source 211 to be focused and introduced into the waveguide 213 without passing through the focal point F2 of the first reflection surface 221.

### 3. Third Embodiment

A third embodiment describes a configuration in which the joining surface is arranged to be slanted with respect to the front-rear direction.

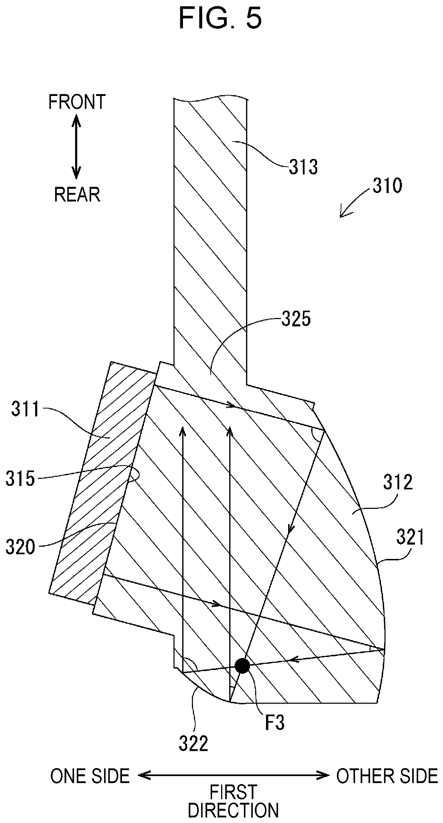

As illustrated in Fig. 5, an ultrasonic wave generating device 310 according to the third embodiment includes an ultrasonic wave generating source 311, an ultrasonic wave focusing part 312, and a waveguide 313. The ultrasonic wave focusing part 312 has a first reflection surface 321 and a second reflection surface 322. The ultrasonic wave generating source 311 has a radiation surface 315, which radiates the ultrasonic waves toward the ultrasonic wave focusing part 312. The ultrasonic wave focusing part 312 has a joining surface 320, which is joined to the ultrasonic wave generating source 311 (more specifically, to the radiation surface 315).

The ultrasonic wave generating device 310 is different from the ultrasonic wave generating device 10 according to the first embodiment mainly in having the joining surface 320, which is slanted. The other elements of the ultrasonic wave generating device 310, excluding the second reflection surface 322, are the same in function as those of the first embodiment, with slight adjustments due to the provision of the slanted second reflection surface 222 to the ultrasonic wave generating device 310.

The joining surface 320 is slanted toward the one side in the first direction while extending rearward. The radiation surface 315 is slanted in the same manner as the joining surface 320. The ultrasonic waves radiated from the radiation surface 315 are reflected by the first reflection surface 321 and are focused toward a focal point F3 of the first reflection surface 321. The focal point F3 of the first reflection surface 321 is the same as that of the second reflection surface 322. The ultrasonic waves reflected by the first reflection surface 321 pass through the focal point F3 of the first reflection surface 321, then reflected by the second reflection surface 322, and introduced as plane waves into an introduction part 325 of the waveguide 313.

In the ultrasonic wave generating device 310 according to the third embodiment, the joining surface 320 and the ultrasonic wave generating source 311 can be arranged to be slanted with respect to the front-rear direction.

### 4. Fourth Embodiment

A fourth embodiment describes a configuration in which a plurality of ultrasonic wave generating sources are arranged side by side in the front-rear direction. The following description of the fourth embodiment mainly focuses on differences from the ultrasonic wave generating device according to the third embodiment. In the fourth embodiment, elements that are the same as those of the third embodiment are denoted by the same reference signs as above, and detailed description of such elements is omitted.

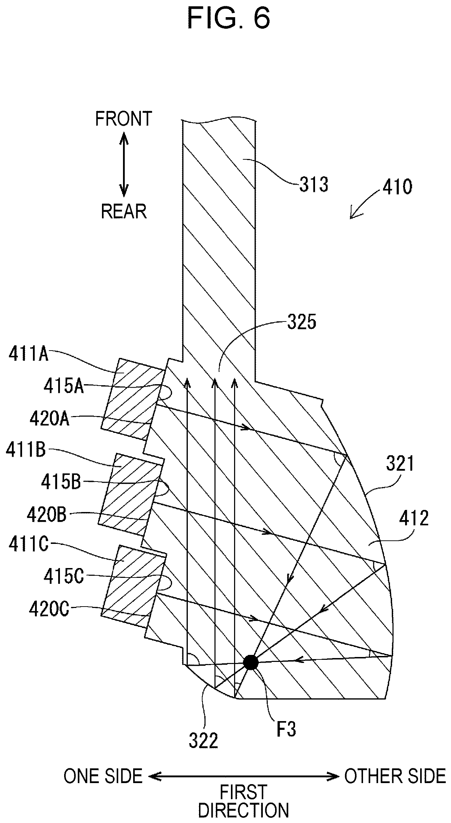

As illustrated in Fig. 6, an ultrasonic wave generating device 410 according to the fourth embodiment includes a plurality of ultrasonic wave generating sources 411A, 411B, and 411C; an ultrasonic wave focusing part 412; and a waveguide 313. The ultrasonic wave generating source 411A has a radiation surface 415A. The ultrasonic wave generating source 411B has a radiation surface 415B. The ultrasonic wave generating source 411C has a radiation surface 415C. The ultrasonic wave focusing part 412 has a first reflection surface 321, a second reflection surface 322, and joining surfaces 420A, 420B, and 420C. The joining surface 420A is joined to the radiation surface 415A of the ultrasonic wave generating source 411A. The joining surface 420B is joined to the radiation surface 415B of the ultrasonic wave generating source 411B. The joining surface 420C is joined to the radiation surface 415C of the ultrasonic wave generating source 411C.

The ultrasonic wave focusing part 412 is different from the ultrasonic wave focusing part 312 according to the third embodiment mainly in having the joining surfaces 420A, 420B, and 420C. The joining surfaces 420A, 420B, and 420C are each slanted with respect to the front-rear direction. The joining surfaces 420A, 420B, and 420C are each slanted toward the one side in the first direction while extending rearward. The joining surfaces 420A, 420B, and 420C are continuous with one another with steps therebetween, and are arranged side by side in the front-rear direction. The ultrasonic wave generating sources 411A, 411B, and 411C joined to the respective joining surfaces 420A, 420B, and 420C are also arranged side by side in the front-rear direction.

The radiation surfaces 415A, 415B, and 415C are slanted in the same manner as the joining surfaces 420A, 420B, and 420C. The ultrasonic waves radiated from the radiation surfaces 415A, 415B, and 415C are reflected by the first reflection surface 321, and are focused toward the focal point F3 of the first reflection surface 321. The focal point F3 of the first reflection surface 321 is the same as that of the second reflection surface 322. The ultrasonic waves reflected by the first reflection surface 321 pass through the focal point F3 of the first reflection surface 321, then reflected by the second reflection surface 322, and introduced as plane waves into the introduction part 325 of the waveguide 313.

In the ultrasonic wave generating device 410 according to the fourth embodiment, the joining surfaces 420A, 420B, and 420C and the ultrasonic wave generating sources 411A, 411B, and 411C can be arranged to be slant with respect to the front-rear direction. Furthermore, the joining surfaces 420A, 420B, and 420C are continuous with one another with steps therebetween, and are arranged side by side in the front-rear direction. Accordingly, the ultrasonic wave generating sources 411A, 411B, and 411C joined to the respective joining surfaces 420A, 420B, and 420C are also arranged side by side in the front-rear direction. Therefore, it is possible to suppress the expansion in the first direction of the joining surfaces 420A, 420B, and 420C and the ultrasonic wave generating sources 411A, 411B, and 411C.

### 5. Fifth Embodiment

A fifth embodiment describes a configuration in which the ultrasonic waves radiated from the radiation surface travel through an area on the rear side relative to the second reflection surface.

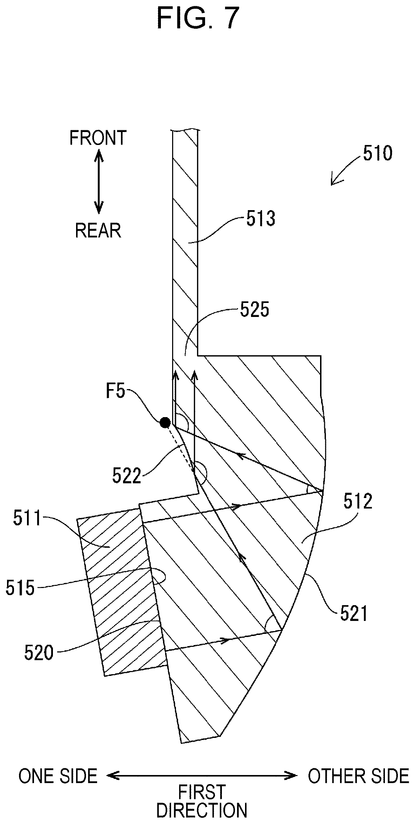

As illustrated in Fig. 7, an ultrasonic wave generating device 510 according to the fifth embodiment includes an ultrasonic wave generating source 511, an ultrasonic wave focusing part 512, and a waveguide 513. The ultrasonic wave generating source 511 has a radiation surface 515, which radiates the ultrasonic waves toward the ultrasonic wave focusing part 512. The ultrasonic wave focusing part 512 has a joining surface 520, which is joined to the radiation surface 515 of the ultrasonic wave focusing part 512. The ultrasonic wave focusing part 512 has a first reflection surface 521 and a second reflection surface 522.

The first reflection surface 521 reflects toward the second reflection surface 522 the ultrasonic waves generated by the ultrasonic wave generating source 511. The second reflection surface 522 reflects toward an introduction part 525 of the waveguide 513 the ultrasonic waves reflected by the first reflection surface 521. The first reflection surface 521 and the second reflection surface 522 are arranged such that the ultrasonic waves reflected by the second reflection surface 522 are reflected as plane waves and introduced into the introduction part 525 of the waveguide 513.

The introduction part 525 and the second reflection surface 522 are arranged to be aligned in the front-rear direction. The introduction part 525 is arranged on the front side relative to the second reflection surface 522. The second reflection surface 522 is arranged facing the other side in the first direction. The first reflection surface 521 is arranged on the other side in the first direction relative to the second reflection surface 522. The first reflection surface 521 is arranged facing the one side in the first direction. The joining surface 520 is arranged on the one side in the first direction relative to the first reflection surface 521. The radiation surface 515 joined to the joining surface 520 is also arranged on the one side in the first direction relative to the first reflection surface 521. The radiation surface 515 faces the first reflection surface 521 with an area located on the rear side relative to the second reflection surface 522 interposed therebetween. A focal point F5 of the second reflection surface 522 is the same as that of the first reflection surface 521.

The ultrasonic waves radiated from the radiation surface 515 travel through the area located on the rear side relative to the second reflection surface 522, and are reflected by the first reflection surface 521. The first reflection surface 521 reflects toward the second reflection surface 522 the ultrasonic waves radiated from the radiation surface 515. The second reflection surface 522 reflects toward the introduction part 525 of the waveguide 513 the ultrasonic waves reflected by the first reflection surface 521.

The ultrasonic wave generating device 510 according to the fifth embodiment can cause the ultrasonic waves radiated from the radiation surface 515 to travel through the area located on the rear side relative to the second reflection surface 522.

### 6. Sixth Embodiment

The first embodiment has described a configuration in which both the ultrasonic wave focusing part and the waveguide each have a shape extending in the second direction. In contrast, a sixth embodiment describes a configuration in which the ultrasonic wave focusing part extends in the second direction, whereas the waveguide is arranged to be intermittent in the second direction. In the sixth embodiment, elements that are the same as those of the first embodiment are denoted by the same reference signs as above, and detailed description of such elements is omitted.

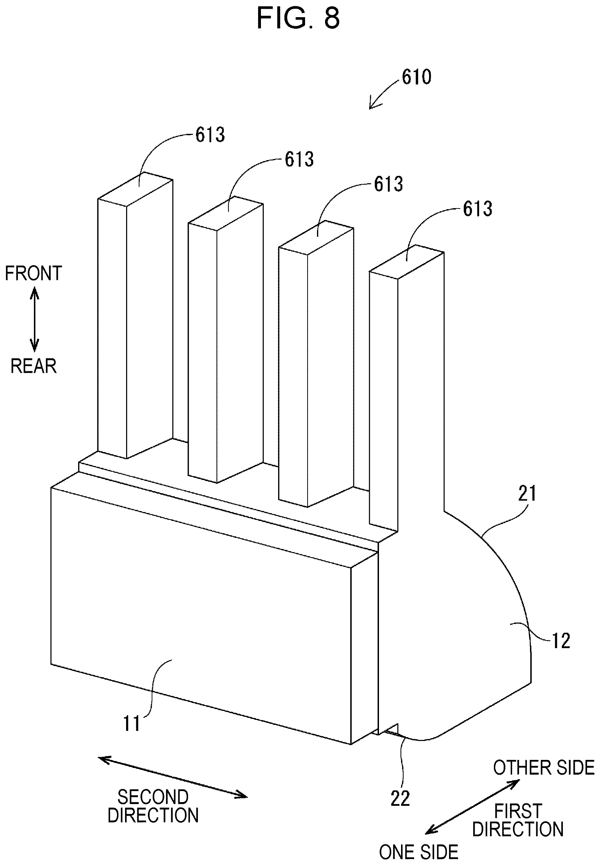

As illustrated in Fig. 8, an ultrasonic wave generating device 610 according to the sixth embodiment includes an ultrasonic wave generating source 11, an ultrasonic wave focusing part 12, and a waveguide 613.

The waveguide 613 has a columnar or needle shape. The waveguide 613 has a shape extending frontward from the front end of the ultrasonic wave focusing part 12. A plurality of waveguides 613 are arranged at intervals from one another in the second direction.

The plane waves generated at the second reflection surface 22 are introduced into each of the waveguides 613 and are transmitted by the waveguides 613.

In the ultrasonic wave generating device 610 according to the sixth embodiment, the ultrasonic waves focused by the ultrasonic wave focusing part 12 can be transmitted by each of the plurality of waveguides 613 arranged at intervals from one another in the second direction.

### 7. Seventh Embodiment

A seventh embodiment describes a configuration in which a flow path is provided inside the waveguide. In the seventh embodiment, elements that are the same as those of the first embodiment are denoted by the same reference signs as above, and detailed description of such elements is omitted.

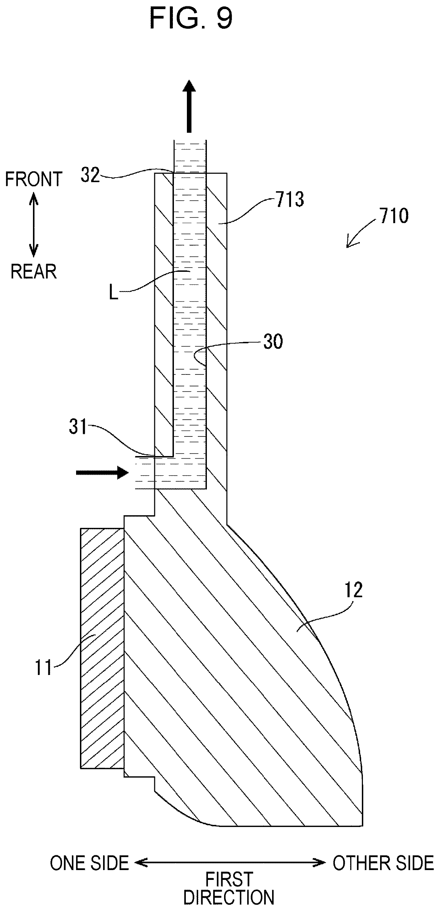

As illustrated in Fig. 9, an ultrasonic wave generating device 710 according to the seventh embodiment includes an ultrasonic wave generating source 11, an ultrasonic wave focusing part 12, and a waveguide 713. A flow path 30 is provided inside the waveguide 713. The waveguide 713 is different in having the flow path 30 from the waveguide 13 according to the first embodiment, but is the same in other points.

The waveguide 713 has a cuboidal shape, as with the waveguide 13 described in the first embodiment. The flow path 30 has one end 31, which is open at the outer surface of the waveguide 713. The flow path 30 has an other end 32, which is arranged at the position on the front side relative to the one end 31 and is open at the distal end of the waveguide 713. Liquid L flows inside the flow path 30. The liquid L flows into the flow path 30 from the one end 31, flows frontward inside the flow path 30, and is discharged from the other end 32. The liquid L is supplied to fill the flow path 30. The flow path 30 may be wide in the second direction, or a plurality of flow paths 30 may be provided at intervals from one another in the second direction.

In the ultrasonic wave generating device 710 according to the seventh embodiment, the liquid L flows through the flow path 30, and the ultrasonic waves are transmitted through the liquid L, whereby the efficiency of transmission of the ultrasonic waves can be improved.

### 8. Eighth Embodiment

An eighth embodiment describes a configuration in which the waveguide has a hollow filled with liquid. In the eighth embodiment, elements that are the same as those of the first embodiment are denoted by the same reference signs as above, and detailed description of such elements is omitted.

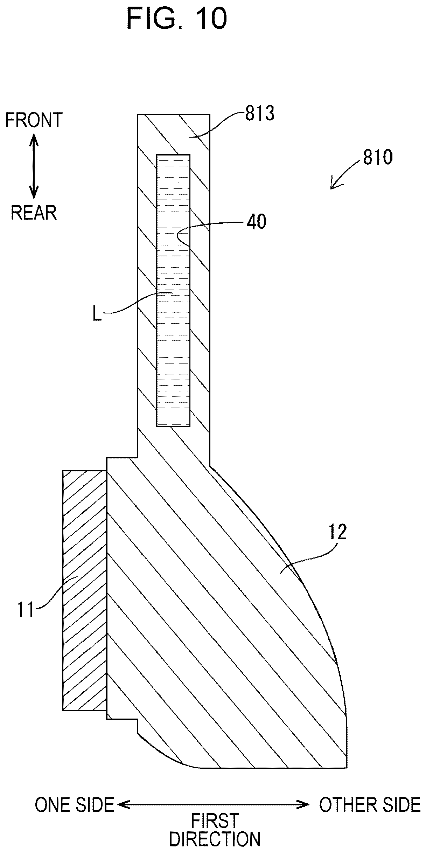

As illustrated in Fig. 10, an ultrasonic wave generating device 810 according to the eighth embodiment includes an ultrasonic wave generating source 11, an ultrasonic wave focusing part 12, and a waveguide 813. The waveguide 813 has a cuboidal shape, as with the waveguide 13 described in the first embodiment. The waveguide 813 has a hollow 40. The waveguide 813 is different in having the hollow 40 from the waveguide 13 according to the first embodiment, but is the same in other points.

The hollow 40 is hermetically closed. The hollow 40 is filled with liquid L. The liquid L may be gel. The method of feeding the liquid L into the hollow 40 is not limited. For example, the waveguide 813 may have a hole for exposing the hollow 40, and the liquid L may be fed into the hollow 40 through the hole. The hole is to be closed after the feeding. Alternatively, the waveguide 813 may have a lid for exposing the hollow 40, and the liquid L may be fed into the hollow 40 with the lid open. The hollow 40 may be wide in the second direction, or a plurality of hollows 40 may be provided at intervals from one another in the second direction.

In the ultrasonic wave generating device 810 according to the eighth embodiment, the ultrasonic waves are transmitted through the liquid L filling the hollow 40, whereby the efficiency of transmission of the ultrasonic waves can be improved.

### <Other Embodiments>

The present invention is not limited to the embodiments described above and illustrated in the drawings. For example, the following embodiments are also within the technical scope of the present invention. Furthermore, various features of the above and following embodiments may be combined in any way, unless the features combined are contradictory to each other.

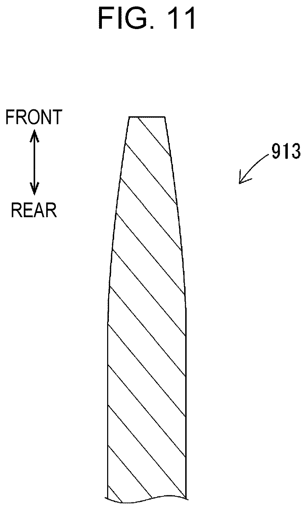

(1) In each of the above embodiments, the ultrasonic wave generating source is directly joined to the ultrasonic wave focusing part, but may be joined to the ultrasonic wave focusing part with another member therebetween.
(2) In the third embodiment, the joining surface is slanted toward one side in the first direction while extending rearward, but may be slanted toward the one side in the first direction while extending frontward.
(3) In the seventh embodiment, the flow path is formed only in the waveguide, but may extend to the ultrasonic wave focusing part. For example, the flow path may be formed to penetrate the ultrasonic wave focusing part in the front-rear direction.
(4) In the seventh embodiment, the other end of the flow path is formed at the distal end of the waveguide, but may be formed at a halfway position on the peripheral surface of the waveguide.
(5) In each of the above embodiments, a plurality of ultrasonic wave generating sources may be provided so as to arranged side by side in the second direction.
(6) In each of the above embodiments, the shape of the distal end portion of the waveguide may be different from the shape of a middle portion (the extended part 26 according to the first embodiment) of the waveguide in the front-rear direction. For example, as with a waveguide 913 illustrated in Fig. 11, the distal end portion may have a tapered shape.

It should be understood that the embodiments disclosed herein are only exemplary in all respects and are not limiting. The scope of the present invention is not limited to the embodiments disclosed herein, and is intended to encompass all changes that are made within the scope defined by the claims or within the scope defined by equivalents of the claims.

### Reference Signs List

- 10: ultrasonic wave generating device
- 11: ultrasonic wave generating source
- 11Z: ultrasonic wave generating source
- 12: ultrasonic wave focusing part
- 12Z: ultrasonic wave focusing part
- 13: waveguide
- 13Z: waveguide
- 15: radiation surface
- 20: joining surface
- 21: first reflection surface
- 21Z: first reflection surface
- 22: second reflection surface
- 22Z: second reflection surface
- 25: introduction part
- 26: extended part
- 30: flow path
- 31: one end
- 32: other end
- 40: hollow
- 210: ultrasonic wave generating device
- 211: ultrasonic wave generating source
- 212: ultrasonic wave focusing part
- 213: waveguide
- 221: first reflection surface
- 222: second reflection surface
- 225: introduction part
- 310: ultrasonic wave generating device
- 311: ultrasonic wave generating source
- 312: ultrasonic wave focusing part
- 313: waveguide
- 315: radiation surface
- 320: joining surface
- 321: first reflection surface
- 322: second reflection surface
- 325: introduction part
- 410: ultrasonic wave generating device
- 411A: ultrasonic wave generating source
- 411B: ultrasonic wave generating source
- 411C: ultrasonic wave generating source
- 412: ultrasonic wave focusing part
- 415A: radiation surface
- 415B: radiation surface
- 415C: radiation surface
- 420A: joining surface
- 420B: joining surface
- 420C: joining surface
- 510: ultrasonic wave generating device
- 511: ultrasonic wave generating source
- 512: ultrasonic wave focusing part
- 513: waveguide
- 515: radiation surface
- 520: joining surface
- 521: first reflection surface
- 522: second reflection surface
- 525: introduction part
- 610: ultrasonic wave generating device
- 613: waveguide
- 710: ultrasonic wave generating device
- 713: waveguide
- 810: ultrasonic wave generating device
- 813: waveguide
- 913: waveguide
- F1: focal point
- F2: focal point
- F3: focal point
- F5: focal point
- L: liquid
- WZ1: ultrasonic wave
- WZ2: ultrasonic wave

## Claims

1. An ultrasonic wave generating device comprising:
an ultrasonic wave generating source that generates ultrasonic waves;
an ultrasonic wave focusing part that focuses the ultrasonic waves generated by the ultrasonic wave generating source; and
a waveguide that transmits the ultrasonic waves focused by the ultrasonic wave focusing part,
wherein the ultrasonic wave focusing part has
a first reflection surface that reflects the ultrasonic waves generated by the ultrasonic wave generating source; and
a second reflection surface that reflects the ultrasonic waves reflected by the first reflection surface,
wherein the first reflection surface and the second reflection surface are arranged such that the ultrasonic waves reflected by the second reflection surface are reflected as plane waves and introduced into an introduction part of the waveguide,
wherein the ultrasonic wave generating source has a radiation surface that radiates the ultrasonic waves toward the ultrasonic wave focusing part,
wherein the ultrasonic wave focusing part has a joining surface joined to the radiation surface,
wherein the introduction part and the second reflection surface are arranged to be aligned in the front-rear direction,
wherein the joining surface is arranged on one side in a first direction, the first direction being a specific direction among directions orthogonal to the front-rear direction, and
wherein the first reflection surface is arranged on an other side in the first direction relative to the joining surface.

2. The ultrasonic wave generating device according to claim 1,
wherein the waveguide has a shape extending in a second direction, the second direction being orthogonal to both the front-rear direction and the first direction.

3. The ultrasonic wave generating device according to claim 1,
wherein a plurality of said waveguides are arranged at intervals from one another in a second direction, the second direction being orthogonal to both the front-rear direction and the first direction.

4. The ultrasonic wave generating device according to any of claims 1 to 3,
wherein a flow path is provided inside the waveguide.

5. The ultrasonic wave generating device according to any of claims 1 to 3,
wherein the waveguide has a hollow, and
wherein the hollow is filled with liquid.

6. The ultrasonic wave generating device according to any of claims 1 to 3,
wherein the joining surface and the first reflection surface are arranged such that the ultrasonic waves radiated from the radiation surface travel through an area between the introduction part and the second reflection surface.

7. The ultrasonic wave generating device according to any of claims 1 to 3,
wherein the radiation surface is formed on a surface of the ultrasonic wave generating source, the surface being located on one side in a thickness direction of the ultrasonic wave generating source, and
wherein a thickness of the ultrasonic wave generating source is smaller than a width of the ultrasonic wave generating source.
